# EUROPEAN PATENT APPLICATION

(11) **EP 4 775 216 A1**
(43) Date of publication of application: **15.07.2026**
(21) Application number: 26151008.5
(22) Date of filing: 09.01.2026
(51) Int. Cl.: A61K 38/17, C07K 16/00, A61K 9/127, A61P 25/00

(54) **PHARMACEUTICAL COMPOSITION COMPRISING AN EXOSOME CONTAINING AN INTEGRIN ANTIBODY FOR USE IN THE TREATMENT OF CENTRAL NERVOUS INJURY DISEASES**

(30) Priority: 10.01.2025 US 202563743757 P
(71) Applicant: China Medical University, Taichung City 406040 (TW)
(72) Inventor: Cho, Der-Yang, 404 Taichung City (TW); Chen, Yi-Wen, 407 Taichung City (TW); Shie, Ming-You, 406 Taichung City (TW); Chiu, Shao-Chih, 404 Taichung City (TW); Huang, Shi-Wei, 81262 Kaohsiung City (TW); Pan, Chih-Ming, 40357 Taichung City (TW); Chen, Mei-Chih, 40878 Taichung City (TW); Hsieh, Ching-Liang, 40442 Taichung City (TW); Chen, Yen, 54048 Nantou City (TW); Chen, Chun-Chung, 40442 Taichung City (TW); Chiu, Cheng-Di, 40866 Taichung City (TW)
(74) Representative: Cabinet Chaillot

(57) **Abstract**

The present invention provides a central nervous system targeting pharmaceutical composition for treating various types of brain injuries. An integrin antibody is bound to a transmembrane protein of an exosome through genetic engineering, and the exosome is used as a carrier to encapsulate a therapeutic agent.

## Description

### FIELD OF INVENTION

A pharmaceutical composition, particularly a genetically engineered pharmaceutical composition that is usable for central nervous system targeting in neurons of brain injury.

### BACKGROUND OF THE INVENTION

Brain injury and neural aging are common degenerative diseases of the central nervous system (CNS) in the brain. The symptoms of these diseases typically appear gradually over time, affecting the motor nervous system, and are currently incurable. Traditionally, drug administration has been used to slow disease progression. However, as neurons degenerate and are lost, and as the blood-brain barrier imposes restrictions, drug treatments gradually lose effectiveness. In the prior arts, exosomes have been utilized to encapsulate drugs, proteins, or nucleic acids, thereby achieving transport and biocompatibility properties, as well as enabling delivery and drug release effects. However, technologies that utilize transmembrane proteins combined with antibodies have not yet matured, and there has been no demonstration of achieving CNS-specific targeting or crossing the blood-brain barrier to reach brain regions. As a result, the development of exosomes in the medical industry remains limited. In view of this, developing pharmaceutical compositions capable of CNS and brain region-specific targeting represents an urgent objective in this field.

### SUMMARY OF THE INVENTION

To develop a targeted pharmaceutical composition capable of specifically binding to the central nervous system and brain regions, the present invention provides an exosome having an integrin antibody bound to a transmembrane protein.

Wherein, the transmembrane protein is selected from the group consisting of CD68, CD81, and CD9.

Wherein, the exosome encapsulates an miRNA-BDNF.

The exosome provided by the present invention is capable of crossing the blood-brain barrier, and can treat or alleviate the progression of brain nerve injury diseases. It can also be used to treat or alleviate the progression of spinal cord injury diseases, as well as to treat motor neurons and promote the activation of neurofilament protein expression.

The present invention provides a central nervous system targeting pharmaceutical composition, comprising an exosome, wherein an integrin antibody is bound to a transmembrane protein of the exosome, wherein the exosome is obtained from a parental cell line after genetic transduction, and at least a portion sequence of the integrin antibody comprises any one of SEQ ID No:1 to SEQ ID No:5.

It also provides a central nervous system targeting pharmaceutical composition for treating or alleviating central nervous system injury diseases, comprising an exosome, wherein an integrin antibody is bound to a transmembrane protein of the exosome, wherein the exosome is obtained from a parental cell line after genetic transduction, and at least a portion sequence of the integrin antibody comprises any one of SEQ ID No:1 to SEQ ID No:5, and the exosome encapsulates a miRNA-BDNF.

The central nervous system injury diseases may comprise stroke, dementia, or spinal cord injury.

The invention also provides the use of the central nervous system targeting pharmaceutical composition for activating neurofilament protein expression, comprising an exosome, wherein an integrin antibody is bound to a transmembrane protein of the exosome, wherein the exosome is obtained from a parental cell line after genetic transduction, and at least a portion sequence of the integrin antibody comprises any one of SEQ ID No:1 to SEQ ID No:5, and the exosome encapsulates a miRNA-BDNF.

### BRIEF DESCRIPTION OF THE DRAWINGS

FIG. 1A is a microscopic image showing the size and shape of the ITExo carrier.
FIG. 1B is a result diagram obtained after Western blot analysis of the ITExo carrier.
FIG. 2A is a schematic diagram of a blood-brain barrier cell culture model.
FIGS. 2B to 2C are analysis diagrams showing the targeting effect of the ITExo carrier in the blood-brain barrier cell culture model.
FIG. 3 A to 3B are inflammatory factor expression analysis diagrams of primary microglia cell in central nervous system injury cell model.
FIGS. 4A to 4B are inflammatory factor expression analysis diagrams of neural cells in the central nervous system injury cell model.
FIG. 5 is an mNSS behavioral test analysis diagram of a brain stroke animal model.
FIG. 6 is a targeting effect analysis diagram of the ITExo carrier in a brain stroke animal model.
FIG. 7A is a TUNEL assay analysis diagram of a brain stroke animal model.
FIG. 7B is a BDNF expression analysis diagram of a brain stroke animal model.
FIG. 8A to 8C are mNSS behavioral test analysis diagrams of a dementia animal model.
FIG. 9 is an analysis diagram showing the targeting effect of the ITExo carrier in a spinal cord injury animal model.
FIG. 10A is a BBB behavioral performance analysis diagram of a spinal cord injury animal model.
FIGS. 10B to 10F are gait analysis system diagrams of a spinal cord injury animal model.
FIGS. 11A to 11D are protein expression analysis diagrams of the injured spinal cord region in a spinal cord injury animal model.

### DETAILED DESCRIPTION OF THE PREFERRED EMBODIMENTS

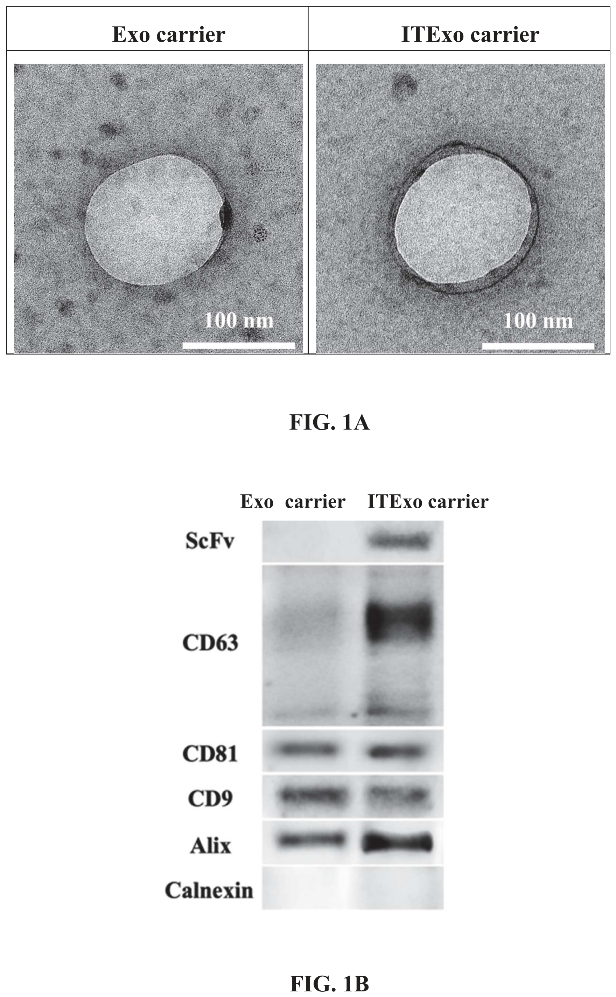

It is known that pathological features of brain injury or neurodegeneration (such as stroke, dementia, amyotrophic lateral sclerosis, and spinal cord injury) include elevated expression of integrins to promote repair. With reference to FIG. 1, to achieve a specific therapeutic effect, the present invention transgenically modifies HEK-293 cells so that at least a portion of a gene sequence in the transgenic HEK-293 cells contains a sequence encoding an integrin antibody (anti-Integrin). Subsequently, an integrin-targeting exosome (hereinafter referred to as the ITExo carrier) secreted by the HEK-293 cells, with an integrin antibody (anti-Integrin) bound on the surface, is obtained.

The ITExo carrier produced in the present invention can specifically bind to neurons affected by brain injury or neurodegeneration through the anti-Integrin, thereby effectively promoting endocytosis in the neurons and avoiding binding of the exosomes to normal cells.

A protein sequence of the integrin antibody (anti-Integrin) comprises an anti-integrin single-chain variable fragment (scFv), selected from any one of SEQ ID No:1 to SEQ ID No:5.

The integrin-targeting exosome is bound to the anti-Integrin through a transmembrane protein such as CD63, CD81, or CD9, and hereinafter the exosome carrier will be described using the CD63 binding site.

The ITExo carrier provided by the present invention can further be loaded with a drug as required, thereby forming a biocompatible pharmaceutical composition. The drug includes, but is not limited to, gene fragments (DNA or RNA), protein sequences, chemical agents, or chemical compounds.

In this embodiment, manufacturing steps of the ITExo carrier include:
Step S1, constructing an integrin antibody plasmid: preparing a vector gene expressing an integrin antibody, and inserting, by genetic engineering techniques, an anti-integrin single-chain variable fragment (scFv) of the integrin antibody into a target gene of the transmembrane protein, such that the fragment gene is combined with the target gene to form the vector gene. The anti-integrin single-chain variable fragment (scFv) is inserted into a gene sequence of CD63 corresponding to the second extracellular loop (large extracellular loop, EC2).

Next, the vector gene is recombined into a plasmid by gene cloning techniques. In this embodiment, the plasmid is a pEXO plasmid (Addgene, Watertown, MA, USA).

Step S2, transfecting the vector gene into a parental cell line and culturing: introducing the plasmid carrying the vector gene into the parental cell line by a transfection technique to form an ITExo cell line, such that the ITExo cell line highly expresses the integrin antibody. The transfection technique includes electroporation, cell squeezing, ultrasound, viral transduction, or chemical transfection. In this embodiment, the transfection technique is lipofection (Lipofectamine 3000, L3000015, Invitrogen, Waltham, MA, USA) is used.

The selection of the parental cell line is not limited and may be made according to the characteristics of the parental cell line. For example, human embryonic kidney 293 (HEK-293) cell line (hereinafter referred to as HEK-293 cell line) exhibits higher transfection efficiency, rapid growth, and simple culture conditions, thereby enabling a large quantity of cells and extracellular vesicles secreted therefrom to be obtained within a short period of time. Alternatively, mesenchymal stem cells (MSCs), which inherently contain abundant growth factors and anti-inflammatory factors, may be used, such that extracellular vesicles secreted therefrom can simultaneously carry abundant growth factors and anti-inflammatory factors to achieve a direct therapeutic effect.

2×10⁸ ITExo cells are distributed in a culture vessel (CelCradle^{®} benchtop bioreactor, ESCO Aster, Singapore) and cultured with 500 mL of Dulbecco's Modified Eagle's Medium (DMEM), wherein the DMEM contains exosome-depleted fetal bovine serum (exosome-depleted FBS, Gibco, Grand Island, NY, USA) and 1% antibiotics (penicillin/streptomycin/amphotericin B solution).

Step S3, collecting the DATEV carrier: after culturing the ITExo cell line for 3 to 4 days, the culture medium from the ITExo cell line is collected and filtered through a 0.22 µm membrane. The filtrate is then concentrated and purified using a tangential flow filtration system with a molecular weight cutoff of 300 kDa (MAP.03-plus TFF System; Lefo Science). The eluate obtained from chromatography is further concentrated through a 30 kDa molecular weight cutoff membrane. Finally, the ITExo carrier released into the culture medium is obtained and resuspended in phosphate-buffered saline (PBS).

The ITExo carrier obtained through the above steps is further analyzed by surface plasmon resonance (SPR), confirming that its affinity for integrin reaches a KD of 3.6 × 10⁻⁹ M.

FIG. 1A demonstrates that the ITExo carrier expresses the integrin antibody (anti-Integrin), and compared with a conventional exosome (Exo carrier), binding of the integrin antibody (anti-Integrin) to the ITExo carrier does not affect its overall size or shape. Western blot analysis verified that the ITExo carrier contains typical exosomal marker proteins (CD63, CD81, CD9, and Alix) and clearly shows enriched expression of CD63 and the integrin antibody (anti-Integrin) in the ITExo carrier.

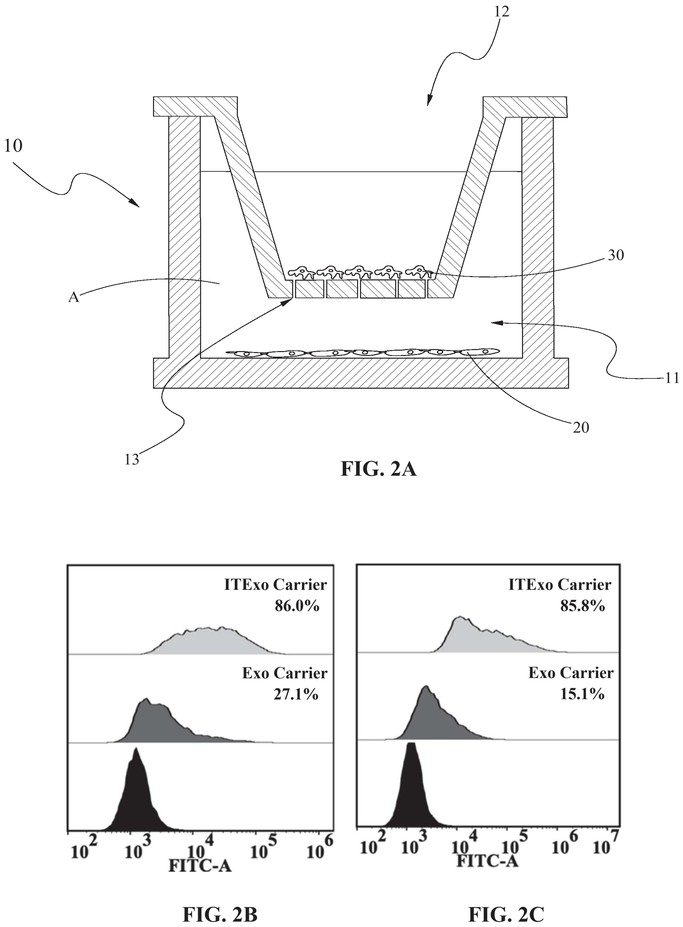

With reference to FIGS. 2A to 2C, to demonstrate that the ITExo carrier can be effectively applied in a biological organism, a blood-brain barrier cell culture model 10 is provided, which is designed to simulate the blood-brain barrier environment in the biological organism and to ensure that the ITExo carrier can cross the blood-brain barrier and perform specific binding to neural cells 20.

The neural cells 20 are derived from human-induced pluripotent stem cells (iPSCs) after induction and differentiation. The procedure includes culturing the iPSCs in PSC neural induction medium (Gibco, A1647801) to induce differentiation into neural stem cells (NSCs), followed by culture in differentiation medium to further differentiate the NSCs into mature neural cells. The differentiation medium includes neurobasal medium (Gibco, 17504044), neuron supplement (B-27^{™} supplement, Gibco, 35050061), and L-alanyl-L-glutamine dipeptide supplement (GlutaMAX^{™} supplement, Gibco, 35050061).

The blood-brain barrier cell culture model 10 includes a first culture chamber 11 and a second culture chamber 12, which are interconnected by multiple perforations 13. Both the first culture chamber 11 and the second culture chamber 12 are filled with a culture medium A. Neural cells 20 are cultured at the bottom of the first culture chamber 11, while primary microglia 30 are positioned at the bottom of the second culture chamber 12 adjacent to the multiple perforations 13, thereby effectively establishing a barrier-like environment of the blood-brain barrier. In subsequent experiments, as required, the Exo carrier and ITExo carrier are separately applied to the second culture chamber 12, and analyses are performed on the primary microglia 30 in the second culture chamber 12 using the neural cells 20 in the first culture chamber 11, to confirm the ability of the ITExo carrier to traverse the second culture chamber 12 and reach the first culture chamber 11.

With reference to FIGS. 2B and 2C, the Exo carrier and the ITExo carrier are labeled with green fluorescence by an immunofluorescence technique, and flow cytometry is used to respectively observe the uptake and presentation of the ITExo carrier in the primary microglia 30 in the second culture chamber 12 and the neural cells 20 in the first culture chamber 11. The results show that the proportion of ITExo carrier internalized into microglia reaches 86.0%, which is significantly higher than the 27.1% observed for the Exo carrier. The same trend is also observed in the neural cells 20, thereby further demonstrating that the ITExo carrier possesses superior targeting capability toward brain neural cells and an enhanced ability to cross the blood-brain barrier (BBB).

Brain-derived neurotrophic factor (BDNF) is a bioactive protein capable of regulating neuronal growth and survival in the brain and promoting synaptogenesis. It is regarded as a highly important neurotrophic protein in the brain, thereby demonstrating the potential of BDNF in the treatment of brain injury and in delaying aging.

To verify the therapeutic effects of BDNF on neurons in the brain, the present invention further employs the ITExo carrier and a conventional exosome (Exo carrier) to encapsulate miRNA-BDNF for the expression of BDNF, followed by in vitro cell experiments and in vivo animal experiments, so as to confirm the specificity and functionality of the ITExo carrier, as well as the effectiveness of BDNF in the treatment of neuronal cells associated with brain injury or neurodegeneration.

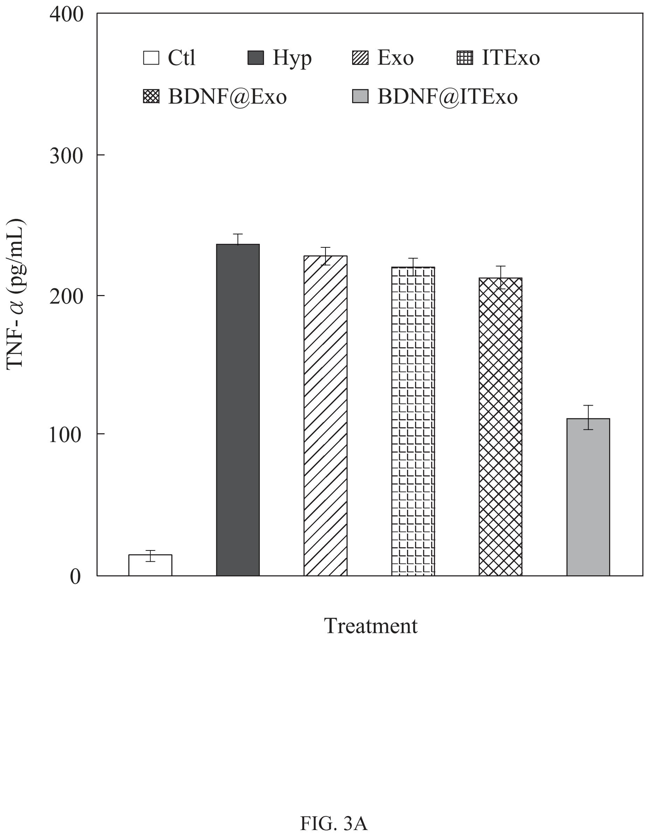

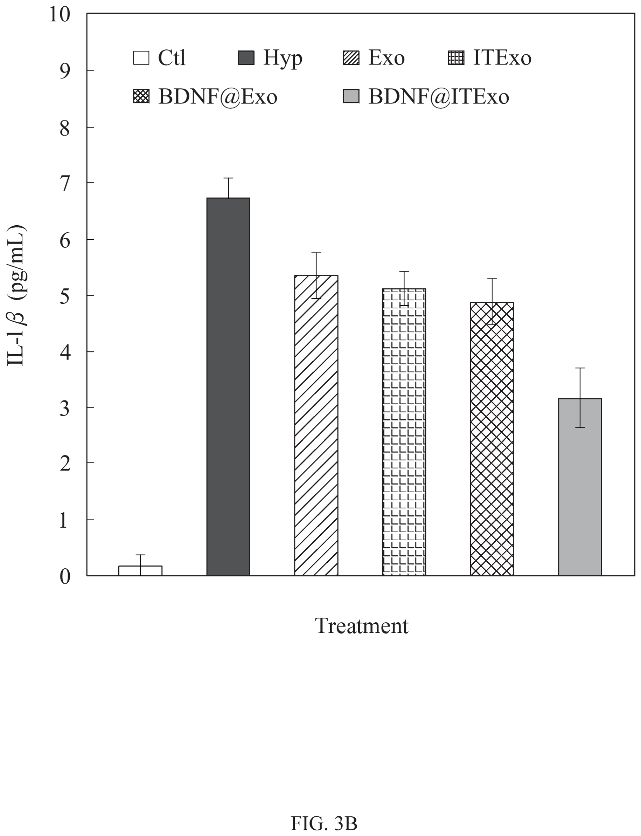

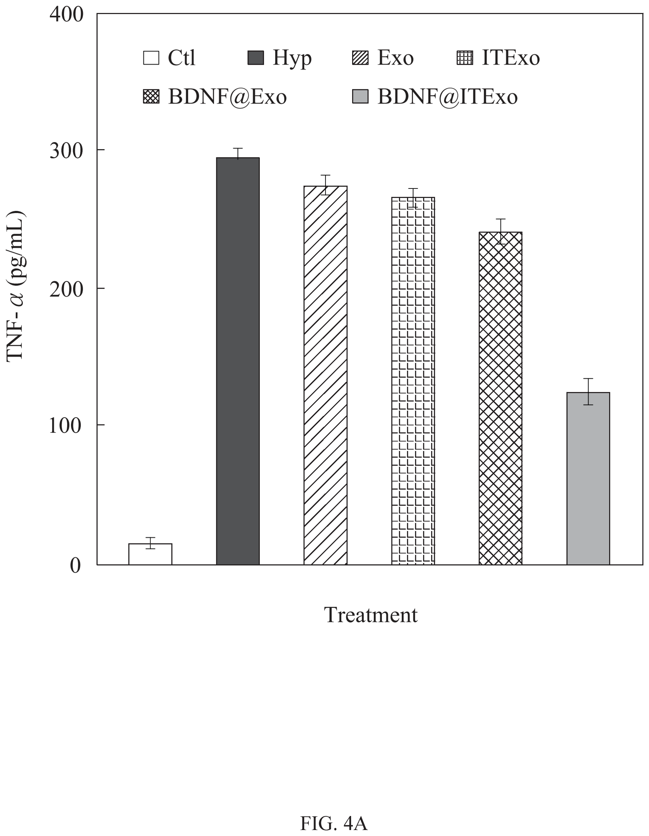

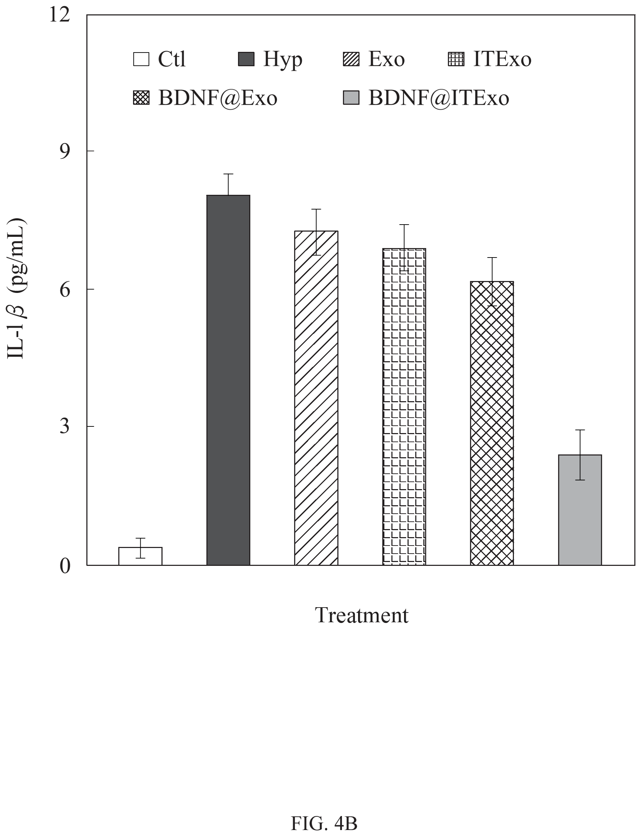

A central nervous system injury model is established by configuring the central nervous system, wherein the aforementioned blood-brain barrier cell culture model 10 is placed at 37 °C and cultured for 48 hours under a hypoxic environment composed of a mixed gas of 92% N₂, 3% O₂, and 5% CO₂, and cellular inflammatory indices of the primary microglia 30(FIG. 3 A and 3B) and the neural cells 20 (FIG. 4 A and 4B) are evaluated, so as to simulate the physiological manifestations of the central nervous system during cerebral stroke.

To verify the therapeutic efficacy of BDNF@ITExo on neural cells in the brain, the present invention further divides the cerebral stroke cell model into the following treatment groups:
Control group 1 (Ctl): a group not subjected to hypoxic culture conditions;
Control group 2 (Hyp): a group subjected to hypoxic culture conditions;
Control group 3 (Exo): a group subjected to hypoxic conditions and co-cultured with Exo carriers;
Control group 4 (ITExo): a group subjected to hypoxic conditions and co-cultured with ITExo carriers;
Control group 5 (BDNF@Exo): a group subjected to hypoxic conditions and co-cultured with Exo carriers loaded with miRNA-BDNF; and
Experimental group 6 (BDNF@ITExo): a group subjected to hypoxic conditions and co-cultured with ITExo carriers loaded with miRNA-BDNF.

In experimental group 6, compared with the other groups, the primary microglia 30and the neural cells 20 are cultured under hypoxic conditions and simultaneously treated with BDNF@ITExo. It can be observed that the expression levels of the inflammatory factors TNF-α and IL-1β in both the primary microglia 30 and the neural cells 20 are significantly reduced.

In addition, the present invention further evaluates the relative expression of a regeneration factor related to regeneration among the groups. In experimental group 6, which is treated with BDNF@ITExo, the expression levels of inflammatory factors are lower compared to the other cerebral stroke cell model groups, and the expression levels of the regeneration factor (such as BDNF, GDNF, MCP-1, and IL-10) are clearly higher than those in the other cerebral stroke cell model groups (not shown in the figures).

The present invention then constructs a brain stroke animal model to test the behavioral changes of the model after treatment with BDNF@ITExo. Blood is collected from the tail of a rat and injected into the rat's brain, followed by a neurological examination (Modified Neurological Severity Score, mNSS) to analyze motor, sensory, reflex, and balance functions of the brain stroke animal model.

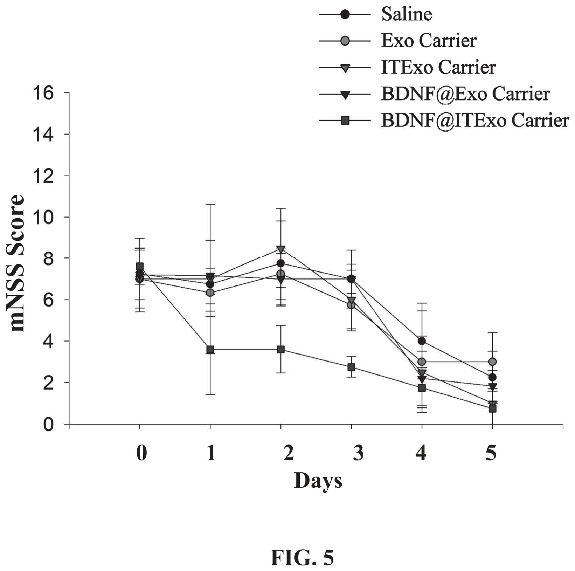

Each of the brain stroke animal models is divided into groups. The brain stroke animal models in each group are intravenously injected for 5 consecutive days with a placebo (Saline), Exo carrier, ITExo carrier, BDNF@Exo carrier, or BDNF@ITExo carrier, each time injecting 100 µL of physiological saline containing 5×10⁹ particles, and mNSS behavioral tests are conducted daily. From the results shown in FIG. 5, it can be seen that in the group treated with BDNF@ITExo, the mNSS scores are significantly lower than those of the other groups (especially on days 2 and 3).

Further, exosomes are labeled in the organs of the brain stroke animal models using the near-infrared lipophilic dye DiR, and the organs are imaged using an IVIS in vivo optical imaging system. The optical signals obtained from the imaging are quantified to compare the absorption of BDNF@ITExo and BDNF@Exo in each organ after administration of BDNF@Exo and BDNF@ITExo, respectively.

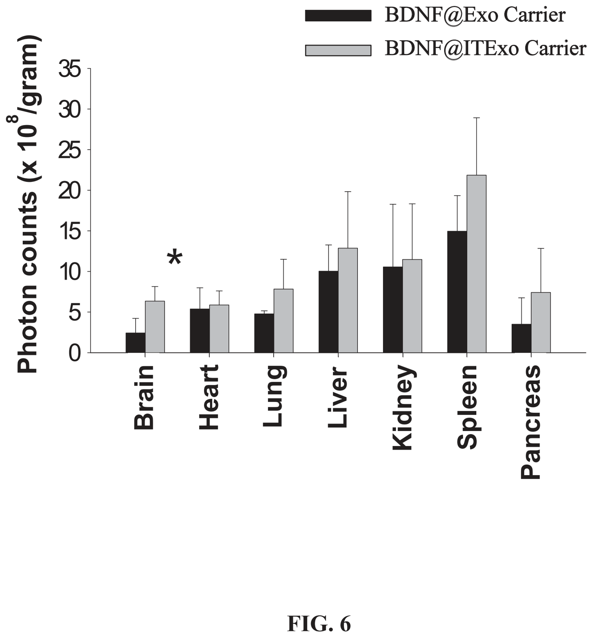

FIG. 6 shows the results after quantification of the optical signals. It can be observed that, in the animal model group administered BDNF@ITExo, a clearly detectable exosome labeling signal is present in the brain. This not only demonstrates the specificity of ITExo but also confirms the ability of ITExo to cross the blood-brain barrier.

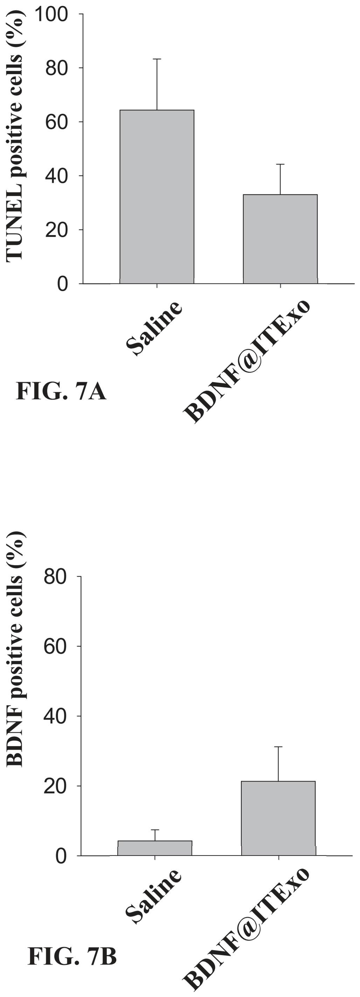

FIG. 7A further observes, in the brain stroke animal model groups respectively administered with the placebo and BDNF@ITExo, rat brain slices subjected to terminal deoxynucleotidyl transferase dUTP nick end labeling (abbreviated as TUNEL assay) to evaluate the proportion of cells exhibiting apoptotic characteristics in the slices. It can be seen that, in the brain stroke animal model group treated with BDNF@ITExo, the proportion of apoptosis-related cells is significantly lower than that in the group treated with the placebo. FIG. 7B then uses immunohistochemistry (IHC) staining technique to respectively detect the protein expression of the regenerative factor BDNF in rat brain slices of the brain stroke animal model groups administered with the placebo and BDNF@ITExo. From the results, it can be determined that, in the brain stroke animal model group treated with BDNF@ITExo, BDNF expression can be clearly detected in the brain slices. Based on the above results, it can be comprehensively evaluated that BDNF is delivered via ITExo across the blood-brain barrier to the brain region with brain injury, thereby reducing the secretion of the inflammatory factors to alleviate the occurrence of inflammatory responses, and increasing the expression of the regenerative factor and BDNF so as to improve the motor function of the brain stroke animal model rats.

Further, to demonstrate that the ITExo carrier can produce the same excellent effects on other brain injury-related diseases, a dementia animal model is further constructed, and behavioral changes of the dementia animal model after treatment with BDNF@ITExo are confirmed. A rat is subjected to posterior brain surgery to induce vascular dementia, and the neurological examination (Modified Neurological Severity Score, mNSS) is used to analyze motor, sensory, reflex, and balance functions of the brain stroke animal model, and a novel object recognition experiment (NOR) is used to confirm the cognitive function of each of the dementia animal models.

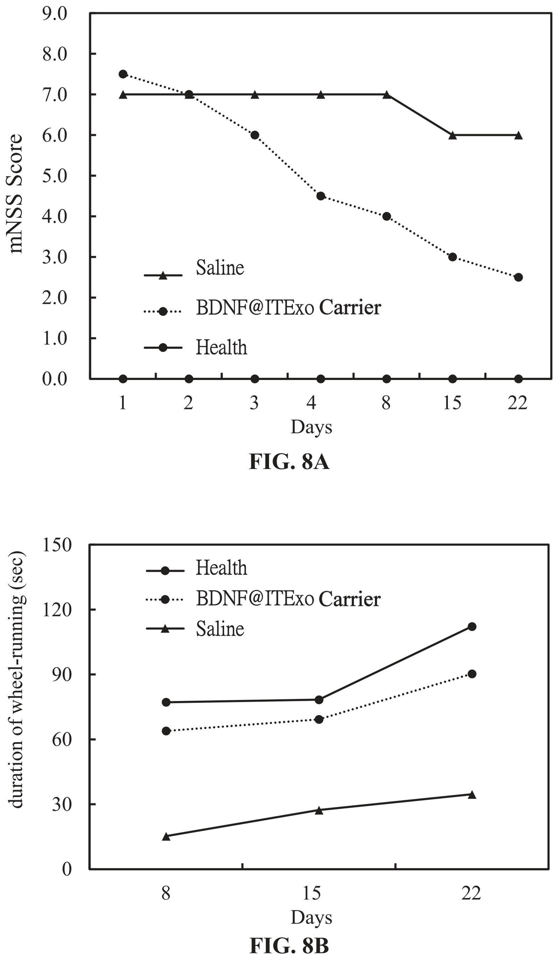

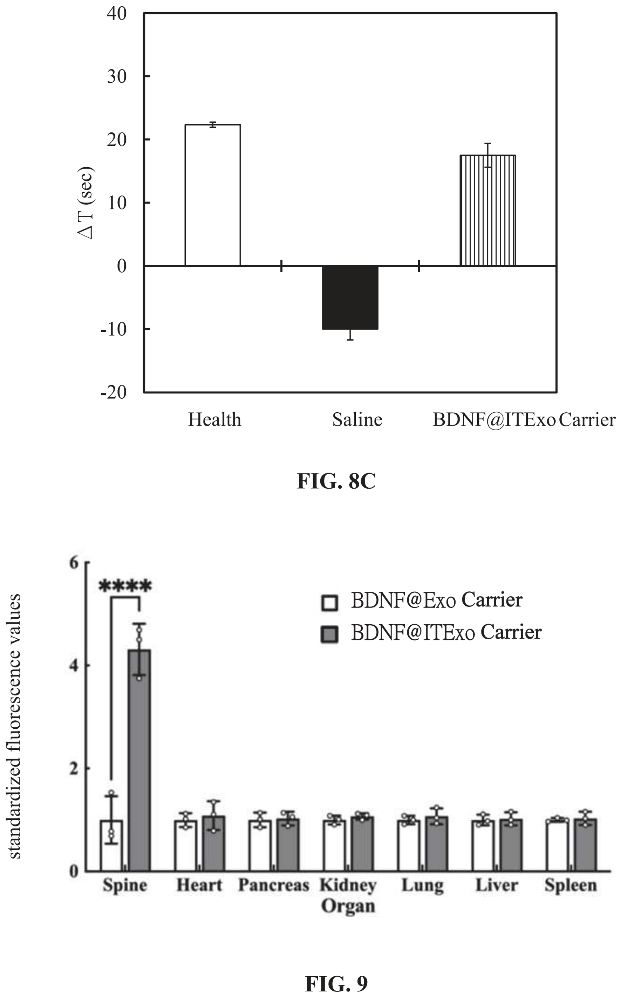

Each of the dementia animal models was grouped, and the brain stroke animal models in each group were administered either the placebo (Saline) or BDNF@ITExo via intravenous injection for 4 consecutive days. Healthy rats (Health) were used as a control group, and daily mNSS behavioral tests and novel object recognition (NOR) tests were performed. As shown in the results of FIG. 8A, in the group treated with BDNF@ITExo, the mNSS scores were significantly lower than those of the other groups starting from day 3. FIGS. 8B and 8C further analyze the rotarod test and the two-object recognition task in the mNSS behavioral test. The rotarod test monitors the duration of wheel-running of the brain stroke animal models on a rotarod for each group. The two-object recognition task evaluates the time spent by the brain stroke animal models exploring a familiar object and a novel object for each group, and the time difference ΔT, calculated as the exploration time of the novel object T2 minus the exploration time of the familiar object T1, is used as an indicator of the ability of the brain stroke animal models to discriminate between new and familiar objects. It can be seen that the group treated with BDNF@ITExo performed better than the dementia animal models administered the placebo (Saline). Notably, in the rotarod test, the duration of wheel-running in the group treated with BDNF@ITExo gradually increased over time, and the overall trend was consistent with that of the control group, indicating that the group treated with BDNF@ITExo still maintained a certain level of motor and learning ability.

In addition, the present invention further constructs a spinal cord injury animal model and confirms the behavioral changes of the spinal cord injury animal model after treatment with BDNF@ITExo. The rat underwent a laminectomy at the T9 vertebra of the thoracic spine, followed by induction of spinal cord contusion using an impactor (parameters set at 250 kilodyne force, 2.5 m/s velocity, and 1.7 mm displacement) to injure the spinal cord and impair its motor function, and motor function was evaluated through the Basso, Beattie, and Bresnahan (BBB) scoring.

Further, exosomes were labeled in various organs of the spinal cord injury animal model using the near-infrared lipophilic dye DiR to compare the absorption of BDNF@ITExo and BDNF@Exo in each organ after administration. In FIG. 9, after standardizing the fluorescence values of BDNF@Exo in each organ, the labeled signal of BDNF@ITExo detected at the spinal cord was approximately five times that of the BDNF@Exo group. This supports the localized expression of integrin at the neural injury site and further confirms the specificity of ITExo, as well as the ability of ITExo to cross the blood-brain barrier.

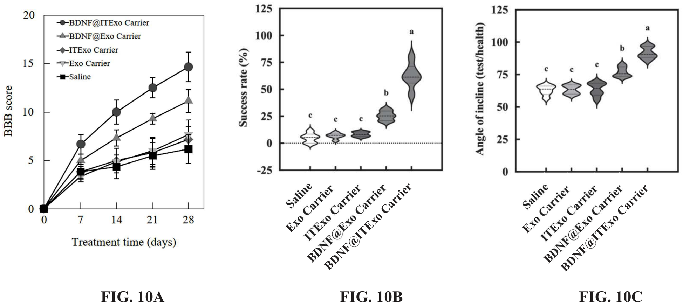

The spinal cord injury animal models were grouped, and each group of the spinal cord injury animal models was administered via intravenous injection for 4 consecutive days with the placebo (Saline), Exo carrier, ITExo carrier, BDNF@Exo carrier, or BDNF@ITExo carrier, with each injection consisting of 100 µL containing 5×10⁹ particles. BBB behavioral tests were conducted on days 7, 14, 21, and 28. As shown in FIG. 10A, the BBB scores of the group treated with BDNF@ITExo were significantly higher than those of the other groups starting from day 7.

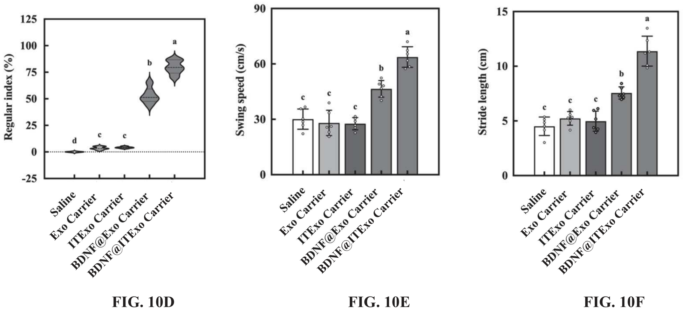

FIG. 10B evaluates gait precision and bilateral coordination of the spinal cord injury animal models of each of the above groups through the grid walk test, by assessing the proportion of correct paw placements to define a success rate. Meanwhile, with reference to FIGS. 10C to 10F, the gait parameters of the spinal cord injury animal models of each group are quantitatively analyzed through a fully automated mouse gait analysis system (Catwalk) to evaluate gait coordination and strength, including the analysis of hind paw landing angle (Angle of incline) of each group with healthy rats as the reference in FIG. 10C; the analysis of gait regularity index of each group normalized with the group given placebo (Saline) as value 0 in FIG. 10D; detection of hind limb swing speed of each group in FIG. 10E; and hind limb stride length of each group in FIG. 10F. From the above results, it can be observed that the group treated with BDNF@ITExo shows the most significant improvements in gait precision, hind limb coordination, and trunk stability, with the success rate higher than the control group and the mBDNF@EV group (p < 0.001) (FIG. 6C, Video 1). This confirms the targeting ability of the ITExo carrier and the significant improvement effect of mBDNF on the spinal cord injury animal models.

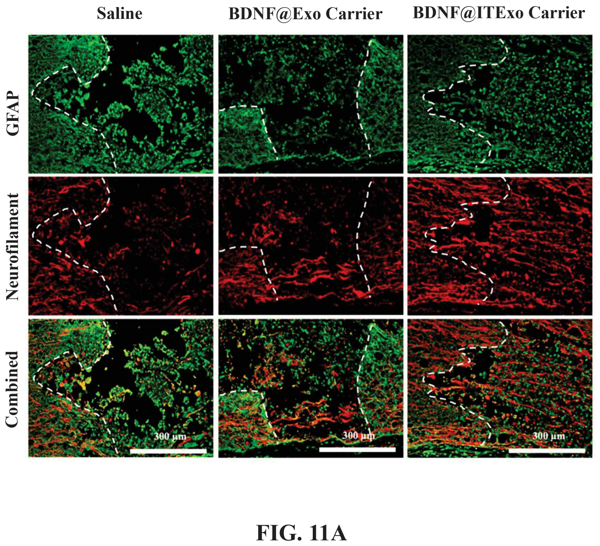

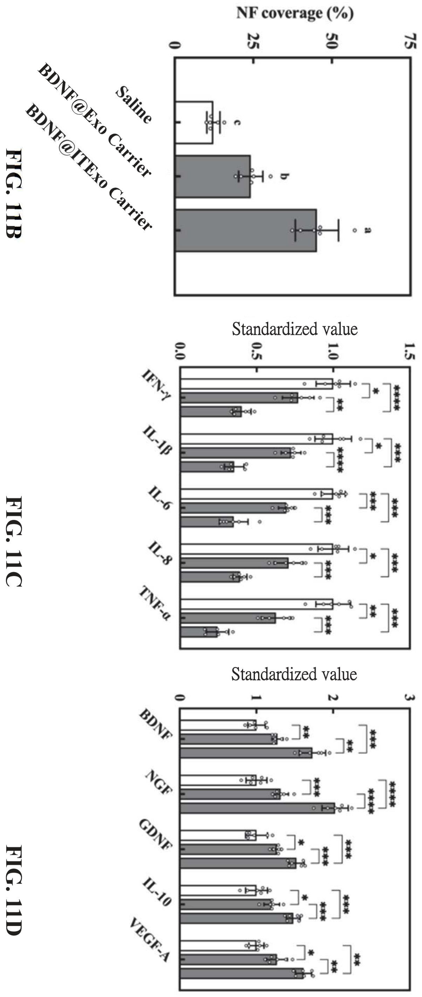

Furthermore, spinal cord sections and immunofluorescence staining were performed at the spinal cord injury sites of the spinal cord injury animal models in the groups respectively treated with placebo (Saline), BDNF@Exo, and BDNF@ITExo. Glial fibrillary acidic protein (GFAP) was used to label astrocytes (green), and neurofilament was used to label neuronal cells (red), to evaluate the growth and repair of nerve fibers at the spinal cord injury sites of the spinal cord injury animal models. From the results in FIG. 11A, it can be observed that in the BDNF@ITExo-treated group, the gap at the spinal cord injury site (white dashed line) caused by the lesion is smaller than that in the other groups, and the fluorescence intensity of astrocytes is weaker than that in the placebo (Saline) and BDNF@Exo groups, indicating that treatment with BDNF@ITExo can reduce the aggregation of astrocytes at the spinal cord injury site. Notably, the spinal cord injury sites of the BDNF@ITExo-treated group also abundantly expressed neurofilament. As shown in FIG. 11B, the red fluorescence area of neurofilament in FIG. 11A at the spinal cord injury site (the area to the right of the white dashed line or the area between the two white dashed lines) was quantified as a percentage of the total area, to evaluate the expression level of neurofilament. The results indicate that nerve fiber growth and repair at the spinal cord injury site in this group are excellent. FIGS. 11C and 11D similarly assessed the expression of inflammatory factors (IFN-y, IL-6, IL-8, IL-1β, and TNF-α) and regenerative factors (such as BDNF, NGF, GDNF, IL-10, and VEGF-A) at the spinal cord injury sites of the above groups, yielding results consistent with the aforementioned experiments.

The ITExo carrier provided by the present invention can load a drug to cross the blood-brain barrier and specifically bind to central nervous system cells after injury. It can effectively reduce the expression of inflammatory factors (IFN-y, IL-1β, IL-6, TNF-α), enhance the expression of regenerative factors (BDNF, NGF, VEGF, IL-10), improve the motor function of animal models with brain or central nervous system injury, and achieve the effect of repairing nerve cells.

## Claims

1. A central nervous system targeting pharmaceutical composition, comprising an exosome, wherein an integrin antibody is bound to a transmembrane protein of the exosome, wherein the exosome is obtained from a parental cell line after genetic transduction, and at least a portion sequence of the integrin antibody comprises any one of SEQ ID No:1 to SEQ ID No:5.

2. The central nervous system targeting pharmaceutical composition according to claim 1, wherein the transmembrane protein is selected from the group comprising CD68, CD81, and CD9.

3. The central nervous system targeting pharmaceutical composition according to claim 2, wherein the exosome encapsulates a miRNA-BDNF.

4. A central nervous system targeting pharmaceutical composition for treating or alleviating central nervous system injury diseases, comprising an exosome, wherein an integrin antibody is bound to a transmembrane protein of the exosome, wherein the exosome is obtained from a parental cell line after genetic transduction, and at least a portion sequence of the integrin antibody comprises any one of SEQ ID No:1 to SEQ ID No:5, and the exosome encapsulates a miRNA-BDNF.

5. The central nervous system targeting pharmaceutical composition for use according to claim 4, wherein the transmembrane protein is selected from the group comprising CD68, CD81, and CD9.

6. The central nervous system targeting pharmaceutical composition for use according to claim 5, wherein the central nervous system injury diseases comprise stroke, dementia, or spinal cord injury.

7. Use of the central nervous system targeting pharmaceutical composition according to claim 1 for activating neurofilament protein expression, comprising an exosome, wherein an integrin antibody is bound to a transmembrane protein of the exosome, wherein the exosome is obtained from a parental cell line after genetic transduction, and at least a portion sequence of the integrin antibody comprises any one of SEQ ID No:1 to SEQ ID No:5, and the exosome encapsulates a miRNA-BDNF.

8. Use of the central nervous system targeting pharmaceutical composition according to claim 7, wherein the transmembrane protein is selected from the group comprising CD68, CD81, and CD9.
